(19)

**Europäisches Patentamt**
**European Patent Office**
**Office européen des brevets**

(11) **EP 1 062 960 B1**

(12) **EUROPÄISCHE PATENTSCHRIFT**

(45) Veröffentlichungstag und Bekanntmachung des Hinweises auf die Patenterteilung:
**15.10.2008 Patentblatt 2008/42**

(51) Int Cl.:
***A61M 1/16*** *(2006.01)*

(21) Anmeldenummer: **00111956.9**

(22) Anmeldetag: **16.06.2000**

(54) **Verfahren zur Bestimmung der Leistungsfähigkeit eines Dialysators, einer Dialysevorrichtung und Dialysevorrichtung zur Durchführung des Verfahrens**

Method for determining the performance of a dialyser and dialysis apparatus therefor

Méthode pour déterminer la performance d'un dialyseur et dispositif de dialyse pour sa mise en oeuvre

(84) Benannte Vertragsstaaten:
**AT BE CH CY DE DK ES FI FR GB GR IE IT LI LU MC NL PT SE**

(30) Priorität: **22.06.1999 DE 19928407**

(43) Veröffentlichungstag der Anmeldung:
**27.12.2000 Patentblatt 2000/52**

(73) Patentinhaber: **Fresenius Medical Care Deutschland GmbH**
**61352 Bad Homburg v.d.H. (DE)**

(72) Erfinder:
 • **Graf, Thomas**
  **97080 Würzburg (DE)**
 • **Gross, Malte, Dr.**
  **97424 Schweinfurt (DE)**
 • **Goldau, Rainer**
  **97440 Werneck (DE)**
 • **Bardorz, Christoph**
  **97228 Rottendorf (DE)**

(74) Vertreter: **Luderschmidt, Schüler & Partner Patentanwälte**
**John-F.-Kennedy-Strasse 4**
**65189 Wiesbaden (DE)**

(56) Entgegenhaltungen:
**DE-A- 3 938 662        DE-A- 19 746 367**
**DE-C- 19 649 775       DE-C- 19 806 900**

 • **SIGDELL J E AND TERSTEEGEN B: "CLEARANCE OF A DIALYZER UNDER VARYING OPERATING CONDITIONS" ARTIFICIAL ORGANS, Bd. 10, Nr. 3, 1986, Seiten 219-225, NEW YORK**

**Beschreibung**

Die Erfindung betrifft ein Verfahren zur Bestimmung der Leistungsfähigkeit eines Dialysators einer Dialysevorrichtung während einer Dialysebehandlung, bei der Blut mit einer vorgegebenen Flußrate durch die Blutkammer eines durch eine semipermeable Membran in die Blutkammer und eine Dialysierflüssigkeitskammer unterteilten Dialysators strömt und Dialysierflüssigkeit mit einer vorgegebenen Flußrate durch die Dialysierflüssigkeitskammer strömt. Darüber hinaus betrifft die Erfindung eine Dialysevorrichtung zur Durchführung des Verfahrens.

Der Stoffaustausch im Dialysator hat sowohl konvektiven als auch diffusiven Charakter. Beim diffusiven Stoffaustausch ist für die betreffende Substanz der Massentransfer pro Zeiteinheit über die Membran proportional dem Konzentrationsgradienten zwischen Blut und Dialysierflüssigkeit; beim konvektiven Strofftransport hängt der Massentransfer von der Filtratmenge ab, da die Konzentration filtrierbarer Substanzen sowohl im Blut als auch im Filtrat gleich ist (Blutreinigungsverfahren, Georg-Thieme-Verlag Stuttgart, New York, 1990, Seiten 11 bis 13).

Da sich das Konzentrationsgefälle während der Dialysebehandlung ständig verringert, kann für die pro Zeiteinheit ausgetauschte Substanzmenge kein fester Zahlenwert angegeben werden. Eine konzentrationsunabhängige Meßgröße für die Leistungsfähigkeit eines Dialysators stellt die Clearance dar.

Die Clearance einer Substanz ist der Teilstrom des Gesamtstroms durch den Dialysator, der von der betreffenden Substanz vollständig befreit ist.

Die Dialysance ist ein weiterer Begriff zur Bestimmung der Leistungsfähigkeit eines Dialysators, bei der auch die Konzentration der Substanz in der Dialysierflüssigkeit Berücksichtigung findet.

Für die Bestimmung der Dialysance D bzw. der Clearance K für eine bestimmte Substanz, beispielsweise Natrium, ergibt sich bei einer Ultrafiltration gleich 0 folgendes.

Die Dialysance D ist gleich dem Verhältnis zwischen dem blutseitigen Massentransport für die betreffende Substanz Qb (cbi - cbo) und der Konzentrationsdifferenz der Substanz zwischen dem Blut und der Dialysierflüssigkeit am jeweiligen Eingang des Dialysators (cbi - cdi).

$$D = Qe \frac{(cbi - cbo)}{cbi - cdi} \qquad (1) \qquad\qquad (1)$$

Aus Gründen der Massenbilanz gilt

$$Qe \bullet (cbi - cbo) = -Qd \bullet (cdi - cdo) \qquad (2)$$

aus (1) und (2) folgt für die Dialysance dialysatseitig:

$$D = -Qd \frac{(cdi - cdo)}{cbi - cdi} \qquad\qquad (3)$$

Dabei sind in (1) bis (3):

Qe = effektiver Blutfluß
Qd = Dialysierflüssigkeitsfluß
cb = Konzentration der Substanz im Lösungsvolumen des Blutes
cd = Konzentration der Substanz in der Dialysierflüssigkeit
i = Eingang des Dialysators
o = Ausgang des Dialysators

Der effektive Blutfluß ist der Fluß des Blutanteils, in dem die am Dialysatorstoffwechsel teilnehmenden Substanzen gelöst sind, d.h. er bezieht sich auf das komplette (wäßrige) Lösungsvolumen für die betreffende Substanz. In Abhängigkeit von der betreffenden Substanz kann dies der Plasmawasserfluß oder der Blutwasserfluß sein.

Für den Fall eines besonderen Stoffwechselausscheidungsprodukts, z.B. Harnstoff, ist cdi gleich null, da diese Substanz bestimmungsgemäß in der frischen Dialysierflüssigkeit nicht vorhanden ist. Man spricht dann nicht mehr von der Dialysance D, sondern von der Clearance C für dieses Stoffwechselprodukt.

Die DE 39 38 662 C2 (EP 0 428 927 A1) beschreibt ein Verfahren zur in-vivo-Bestimmung von Parametern der Hämodialyse, insbesondere der Dialysance, bei dem der Dialysat-Elektrolythtransfer jeweils bei zwei unterschiedlichen Dialysateingangskonzentrationen gemessen wird. Unter der Annahme, daß die Bluteingangskonzentration konstant ist, wird nach dem bekannten Verfahren die Dialysance dadurch bestimmt, daß die Differenz zwischen den Differenzen der Dialysierflüssigkeits-Ionenkonzentration an der Eingangsseite und der Ausgangsseite des Dialysators zum Zeitpunkt der ersten und zweiten Messung ermittelt wird, diese durch die Differenz der Dialysierflüssigkeits-Ionenkonzentration an der Eingangsseite zum Zeitpunkt der ersten Messung und der zweiten Messung geteilt wird und mit dem Dialysierflüssigkeitsfluß multipliziert wird.

Für eine Überwachung des zeitlichen Verlaufs der Dialysance während der Dialysebehandlung erweist sich bei den bekannten Verfahren die verhältnismäßig lange Meßzeit als nachteilig, die darauf zurückzuführen ist, daß nach dem Einstellen der Dialysierflüssigkeit auf die neue Eingangskonzentration sich am Dialysatorausgang erst ein stabiler Zustand einstellen muß, bevor der Meßwert aufgenommen werden kann. Es dauert systembedingt einen gewissen Zeitraum, bis ein Leitfähigkeitssprung am Dialysatoreingang zu stabilen Verhältnissen im Dialysatorausgang führt.

Die DE 197 39 100 C1 beschreibt ein Verfahren zur Bestimmung der maximalen Dialysance während der Dialysebehandlung. Bei dem bekannten Verfahren wird die Dialysierflüssigkeitseingangskonzentration einer bestimmten Substanz in der Dialysierflüssigkeit stromauf der Dialysierflüssigkeitskammer, die Dialysierflüssigkeitsausgangskonzentration der betreffenden Substanz stromab der Dialysierflüssigkeitskammer des Dialysators und die Bluteingangskonzentration der Substanz im Blutstrom stromauf der Blutkammer des Dialysators bestimmt und aus der Dialysierflüssigkeiteingangs- und ausgangskonzentration, der Bluteingangskonzentration sowie dem Blutfluß durch die Blutkammer und dem Dialysierflüssigkeitsfluß durch die Dialysierflüssigkeitskammer die maximale Dialysance bestimmt. Nachteilig ist, daß das bekannte Verfahren nur die Bestimmung der maximalen Dialysance, nicht aber der Dialysance für einen beliebigen Dialysierflüssigkeits- bzw. Blutfluß erlaubt.

Aus der DE 197 46 367 A1 ist ein Verfahren zur in-vivo-Bestimmung von Parametern der Hämodialyse, bspw. der Clearance oder Dialysance bekannt, bei dem in einem Messintervall die Dialysierflüssigkeits- und/oder Blutflussrate ermittelt und die Parameter der Dialyse bei der ermittelten Flussrate bestimmt werden.

Der Artikel von Sigdell und Tersteegen: "Clearance of a Dialyzer Under Varying Operating Conditions" Bd.10, Nr. 3, 1986, Seiten 219 bis 225, New York, beschreibt den mathematischen Zusammenhang zwischen der Clearance einerseits und der Dialysierflüssigkeits- und Blutflussrate andererseits. Der Artikel macht deutlich, dass die Clearance im Wesentlichen nur von der Blutfluss- und Dialysierflüssigkeitsrate abhängig ist, wenn die Ultrafiltration gleich Null ist.

Der Erfindung liegt die Aufgabe zugrunde, ein Verfahren anzugeben, das die Überwachung der Leistungsfähigkeit des Dialysators während der Dialysebehandlung ohne zeitliche Verzögerungen auf einfache Weise ermöglicht, und eine Dialysevorrichtung zu schaffen, die eine Überwachung der Leistungsfähigkeit des Dialysators ohne zeitliche Verzögerungen auf einfache Weise erlaubt. Die Lösung dieser Aufgabe erfolgt mit den Merkmalen des Patentanspruchs 1 bzw. 7.

Das erfindungsgemäße Verfahren beruht darauf, daß die Dialysance und/oder Clearance des Dialysators für beliebige Dialysierflüssigkeits-, und/oder Blutfluß-, und/oder Ultrafiltrationsraten auf der Grundlage der sich bei einer vorgegebenen Dialysierflüssigkeits-, Blutfluß- bzw. Ultrafiltrationsrate einstellenden Clearance und/oder Dialysance bestimmt wird, wobei die sich bei der vorgegebenen Dialysierflüssigkeits-, Blutfluß- bzw. Ultrafiltrationsrate einstellende Clearance und/oder Dialysance in bekannter Weise während der Dialysebehandlung gemessen werden kann. Insofern ist nur eine einzige Messung erforderlich, um den zeitlichen Verlauf der Dialysance und/oder Clearance überwachen zu können.

Wenn die sich bei der vorgegebenen Dialysierflüssigkeits-, Blutfluß- bzw. Ultrafiltrationsrate einstellende Clearance und/oder Dialysance bekannt sein sollte, ist die Messung derselben nicht erforderlich. So kann beispielsweise die Bestimmung der Leistungsfähigkeit des Dialysators für beliebige Dialysierflüssigkeits-, Blutfluß- bzw. Ultrafiltrationsraten auf der Grundlage der für eine bestimmte Dialysierflüssigkeits-, Blutfluß- bzw. Ultrafiltrationsrate vom Hersteller angegebenen Dialysance bzw. Clearance des Dialysators erfolgen.

Auf der Grundlage der sich bei einer bestimmten Dialysierflüssigkeits-, Blutfluß- bzw. Ultrafiltrationsrate einstellenden Clearance und/oder Dialysance kann grundsätzlich der zeitliche Verlauf der Clearance bzw. Dialysance während der gesamten Dialysebehandlung abgeschätzt werden. Zur Erhöhung der Genauigkeit kann die gesamte Dialysebehandlung aber auch in einzelne Zeitabschnitte unterteilt werden, in denen dann die Überwachung auf der Grundlage jeweils einer Messung erfolgt.

Zur Überwachung der Leistungsfähigkeit des Dialysators können die Clearance und/oder Dialysance in Abhängigkeit von der Dialysierflüssigkeits-, Blutfluß- bzw. Ultrafiltrationsrate während der Dialysebehandlung fortlaufend bestimmt werden. Die Ermittlung der effektiven Clearance bzw. Dialysance kann dann durch Mittelwertbildung erfolgen.

Die Dialysierflüssigkeits-, Blutfluß- bzw. Ultrafiltrationsrate kann während der Dialysebehandlung gemessen werden, vorzugsweise werden jedoch die Förderraten der Blutpumpe bzw. Dialysierflüssigkeitspumpe in der arteriellen oder venösen Blutleitung bzw. der Dialysierflüssigkeitszuführ- bzw. abführleitung zur Bestimmung der Flußraten erfaßt. Die Ultrafiltrationsrate ergibt sich durch Differenzbildung der Flüße in den zu- und abführenden Leitungen, sie kann auch durch die Förderrate einer Ultrafiltrationspumpe vorgegeben werden, wie z:B. in der DE 42 39 937 A1 beschrieben ist.

Die Einrichtung zur Bestimmung der Clearance und/oder Dialysance der Dialysevorrichtung weist eine Recheneinheit auf, in der die entsprechenden Parameter der Dialyse berechnet werden.

Das erfindungsgemäße Verfahren bzw. die erfindungsgemäße Vorrichtung ist insbesondere in den Fällen vorteilhaft einsetzbar, bei denen sich die Flußraten aufgrund von Gerätedriften selbständig verändern, ohne daß der Benutzer eine direkte Flußänderung veranlaßt hat.

Zusätzlich führt eine Hämodialysebehandlung zwangsläufig zu einer Veränderung der Blutzusammensetzung während der Behandlung. Insbesondere der wäßrige Anteil wird durch die Ultrafiltration verringert. I. a. ändert sich dadurch der effektive Blutfluß Qe und der Hämatokrit, ohne daß die Pumprate der Blutpumpe von außen - z. B. durch Änderung der Umdrehungszahl der zumeist verwendeten Rollenpumpen - verändert wurde. Da für die Anwendungen der Erfindung jedoch nur die Feststellung der beteiligten Flußraten notwendig ist, können derartige Einflüsse durch geeignete Meßsensoren erfaßt werden.

Hierzu können direkte Flußsensoren vorgesehen sein. Es ist aber auch möglich, Zusammenhänge zwischen der Flußrate und anderen Größen anzuwenden. Als Beispiel sei eine Korrektur der sich aufgrund der Umdrehungszahl ergebenden nominellen Förderrate einer Rollenpumpe durch Berücksichtigung des Ladedrucks genannt.

Auf diese Weise ist es möglich, sehr genaue Angaben über die Leistungsfähigkeit eines Dialysators bzw. einer Dialysebehandlung zu erhalten, ohne die Behandlung durch zu häufige Messungen der Dialysance bzw. Clearance unterbrechen zu müssen. Je nach Meßverfahren und Häufigkeit der Messung können derartige Messungen ansonsten zu einer Beeinträchtigung der eigentlichen Dialysebehandlung führen.

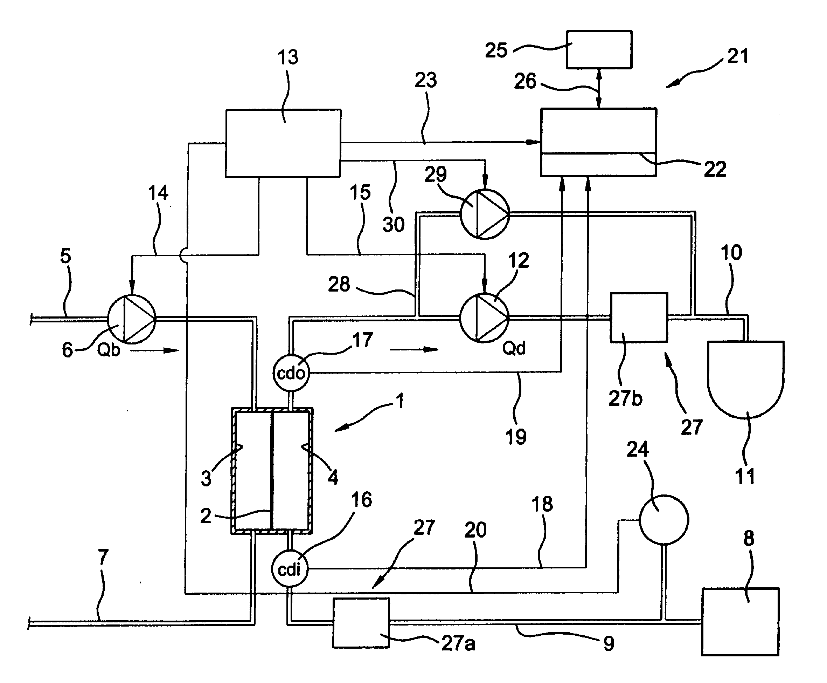

Im folgenden wird eine bevorzugte Ausführungsform der Dialysevorrichtung mit einer Einrichtung zur Bestimmung der Dialysance und/oder Clearance unter Bezugnahme auf die Zeichnung im einzelnen beschrieben, wobei die einzelnen Verfahrensschritte näher erläutert werden.

Die Dialysevorrichtung weist einen Dialysator 1 auf, der durch eine semi-permeable Membran 2 in eine Blutkammer 3 und eine Dialysierflüssigkeitskammer 4 unterteilt ist. An dem Einlaß der Blutkammer 3 ist eine arterielle Blutleitung 5 angeschlossen, in die eine Blutpumpe 6 geschaltet ist. Stromab der Blutkammer führt eine venöse Blutleitung 7 von dem Auslaß der Blutkammer zu dem Patienten.

In einer Dialysierflüssigkeitsquelle 8 wird frische Dialysierflüssigkeit bereitgestellt. Von der Dialysierflüssigkeitsquelle 8 führt eine Dialysierflüssigkeitszuführleitung 9 zu dem Einlaß der Dialysierflüssigkeitskammer 4 des Dialysators 1, während eine Dialysierflüssigkeitsabführleitung 10 von dem Auslaß der Dialysierflüssigkeitskammer zu einem Abfluß 11 führt. In die Dialysierflüssigkeitsabführleitung 10 ist eine Dialysierflüssigkeitspumpe 12 geschaltet.

Zur Bilanzierung der in den Dialysator fließenden bzw. aus dem Dialysator strömenden Flüssigkeit ist eine Bilanziereinrichtung 27 vorgesehen, die über eine Bilanzkammer mit zwei Bilanzkammerhälften 27a,27b verfügt, von denen die erste in die Dialysierflüssigkeitszuführ- 9 und die zweite in die Dialysierflüssigkeitsabführleitung 10 geschaltet ist. Stromauf der Dialysierflüssigkeitspumpe 12 zweigt eine Ultrafiltrationsleitung 28 ab, die stromab der zweiten Bilanzkammer 27b wieder in die Dialysierflüssigkeitabführleiutng mündet. In die Ultrafiltrationsleitung 28 ist eine Ultrafiltrationspumpe 29 geschaltet, deren Förderrate die Ultrafiltrationsrate vorgibt.

Die Dialysevorrichtung verfügt über eine Steuereinheit 13, die mit der Blutpumpe 6, der Dialysierflüssigkeitspumpe 12 und der Ultrafiltrationspumpe 29 über Steuerleitungen 14, 15, 30 verbunden ist. Die Steuereinheit 13 stellt eine bestimmte Förderrate für die Blutpumpe 6, die Dialysierflüssigkeitspumpe 12 sowie die Ultrafiltrationspumpe 28 ein, die vom Benutzer vorgegeben und während der Dialysebehandlung verändert werden kann.

In der Dialysierflüssigkeitszuführleitung 9 am Einlaß der Dialysierflüssigkeitskammer 4 ist ein Leitfähigkeitssensor 16 zur Bestimmung der Dialysierflüssigkeitseingangskonzentration Cdi einer bestimmten Substanz in der Dialysierflüssigkeit stromauf der Dialysierflüssigkeitskammer und ein Leitfähigkeitssensor 17 in der Dialysierflüssigkeitsabführleitung 10 am Auslaß der Dialysierflüssigkeitskammer 4 angeordnet, der die sich während der Dialysebehandlung einstellende Dialysierflüssigkeitsausgangskonzentration Cdo der betreffenden Substanz in der Dialysierflüssigkeit stromab des Dialysators mißt.

Die Meßwerte der Leitfähigkeitssensoren 16, 17 werden über Signalleitungen 18, 19 einer Einrichtung 21 zur Bestimmung der Dialysance D und Clearance C, zugeführt, die eine Recheneinheit 22 aufweist. Die Recheneinheit 22 wird vorzugsweise von einem Mikroprozessor gebildet, der in den bekannten Dialysevorrichtungen ohnehin vorhanden ist. Über eine zu der Steuereinheit 13 führende Datenleitung 23 erfaßt die Einrichtung 21 zur Bestimmung der Dialysance und der Clearance die den Blutfluß Qb bzw. den Dialysierflüssigkeitsfluß Qd bzw. die Ultrafiltrationsrate vorgebenden Förderraten der Blutpumpe 6 bzw. Dialysierflüssigkeitspumpe 12 bzw. Ultrafiltrationspumpe 29.

Zur Veränderung der Na-Konzentration der Dialyierflüssigkeit stromauf des Dialysators 1 ist eine weitere Einrichtung 24 vorgesehen. Mit der Einrichtung 24 kann die in den Dialysator fließende Dialysierflüssigkeit bezüglich ihrer Zusammensetzung verändert werden. Über eine Steuerleitung 25 ist die Einrichtung 24 mit der Steuereinheit 13 verbunden.

Die Dialysevorrichtung kann noch über weitere Komponenten, z. B. eine Tropfkammer, Sperrorgange etc. verfügen, die der besseren Übersichtlichkeit halber jedoch nicht dargestellt sind.

Die Leistungsfähigkeit des Dialysators kann wie folgt überwacht werden. Für eine zu Beginn der Dialysebehandlung vorgegebene Dialysierflüssigkeits-, Blutfluß-und Ultrafiltrationsrate Qd1, Qb1, Qf1 werden die Dialysierflüssigkeitseingangs- bzw. -ausgangskonzentration Cdi1,Cdo1 mit den Leitfähigkeitssensoren 16, 17 gemessen. Daraufhin steuert die Steuereinheit 22 die Einrichtung 24 derart an, daß die Na-Konzentration der Dialysierflüssigkeit stromauf des Dialysators erhöht wird, wobei wieder die Dialysierflüssigkeitseingangs- und -ausgangskonzentration Cdi2, Cdo2 mit den Leitfähigkeitssensoren 16, 17 gemessen werden. Aus den gewonnenen Meßwerten wird dann in der Recheneinheit die Clearance K1 bzw. Dialysance bei der vorgegebenen Dialysierflüssigkeits-, Blutfluß- und Ultrafiltrationsrate Qd1,Qb1 Qf1 nach der folgenden Gleichung berechnet:

$$K1 = \left(1 - \frac{Cdo2 - Cdo1}{Cdi2 - Cdi1}\right) \cdot \left(Qd1 + Qf1\right) \qquad (4)$$

Unter der Voraussetzung, daß während der Dialysebehandlung nur die Dialysierflüssigkeits-, Blutfluß- bzw. Ultrafiltrationsrate verändert wird, berechnet die Recheneinheit aus der ermittelten Clearance K1 bzw. Dialysance und der vorgegebenen Blutflußrate Qb1 bzw. Ultrafiltrationsrate Qf1 zunächst den diffusiven Anteil D1 an der Clearance bzw. Dialysance wie folgt:

$$D1 = \frac{K1 - Qf1}{1 - Qf1 / Qe1} \qquad (5)$$

Der effektive Blutfluß Qe berechnet sich dabei aus dem der Förderrate der Blutpumpe entsprechenden absoluten Blutfluß Qb wie folgt:

$$Qe = Qb\left(1 - \frac{HCT}{100}\right)Fp \qquad (6)$$

wobei HCT der Hämatokrit [%] und Fp die Plasmawasserfraktion ist.

Nach der Bestimmung des diffusiven Anteils D1 der Dialysance bzw. Clearance berechnet die Recheneinheit die diffusive Dialysance D(Qd(t),Qe(t)) für beliebige Dialysierflüssigkeits-, Blutfluß- bzw. Ultrafiltrationsraten Qd(t), Qe(t), Qf(t) nach den folgenden Gleichungen:

$$D(Qd(t),Qe(t)) = Qe(t) \cdot \left(1 - \exp\left(\frac{Qd(t)}{Qd1}ln\left(1 - \frac{DQecorr}{Qd(t)}\right)\right)\right) \qquad (7)$$

mit

$$DQecorr = Qd1\left(1 - \exp\left(\frac{Qe(t)}{Qe1}ln\left(1 - \frac{D1}{Qd1}\right)\right)\right) \qquad (8)$$

Die Berechnung der diffusiven Dialysance D(Qd(t),Qe(t)) erfolgt immer dann, wenn die Dialysierflüssigkeits- oder Blutflußrate, die mit der Förderrate der Dialysierflüssigkeits- bzw. Blutpumpe 12, 6 korreliert, verändert wird. Nach der folgenden Gleichung berechnet die Recheneinheit aus der diffusiven Dialysance D(Qd(t),Qe(t)) die Summe von diffusiver und konvektiver Dialysance bzw. Clearance K(Qd(t),Qe(t),Qf(t):

$$K(Qd(t), Qe(t), Qf(t)) = D((Qd(t), Qe(t)))\left(1 - \frac{Qf(t)}{Qe(t)}\right) + Qf(t) \qquad (9)$$

Der zeitliche Verlauf der Clearance bzw. Dialysance wird während der Dialysebehandlung ermittelt. In der Recheneinheit wird aus den einzelnen Werten für die Clearance durch Mittelwertbildung die effektive Clearance $K_{eff}$ berechnet.

Ist der zeitliche Verlauf der Clearance während der Dialysebehandlung mit der Behandlungsdauer T bekannt, kann die Berechnung der effektiven Clearance $K_{eff}$ auch durch Integration nach der folgenden Gleichung erfolgen:

$$Keff = \frac{1}{T} \int_0^T K(t)dt \qquad (10)$$

Während die Werte für K(t) auf verschiedenen Meßwerten beruhen können, ist es nach der Erfindung möglich, Messungen für solche t auszusetzen, bei denen K(t) nach Gleichung (9) mit ausreichender Genauigkeit bestimmt werden kann.

Die Werte für die Clearance bzw. Dialysance sowie die effektiven Werte werden auf einer Anzeigeeinheit 25 angezeigt, die über eine Datenleitung 26 mit der Recheneinheit 22 in Verbindung steht. Damit kann die Reinigungsleistung des Dialysators während der Dialysebehandlung laufend überwacht werden.

**Patentansprüche**

1. Verfahren zur Bestimmung der Leistungsfähigkeit eines Dialysators einer Dialysevorrichtung während einer Dialysebehandlung, bei der Blut mit einer vorgegebenen Flußrate durch die Blutkammer (3) eines durch eine semipermeable Membran (2) in die Blutkammer und eine Dialysierflüssigkeitskammer (4) unterteilten Dialysators (1) strömt und Dialysierflüssigkeit mit einer vorgegebenen Flußrate durch die Dialysierflüssigkeitskammer strömt, **dadurch gekennzeichnet,**
   **daß** die Flußrate des Blutes Qb und der Dialysierflüssigkeit Qd durch die Blutkammer (3) bzw. Dialysierflüssigkeitskammer (4) des Dialysators (1) und die Ultrafiltrationsrate Qf während der Dialysebehandlung ermittelt wird, und daß auf der Grundlage der sich bei einer vorgegebenen Blutflußrate Qb1, Dialysierflüssigkeitsrate Qd1 und Ultrafiltrationsrate Qf1 einstellenden Clearance K1 und/oder Dialysance die Clearance und/oder Dialysance D(Qd(t),Qe(t),Qf(t)) bei der ermittelten Blutflußrate Qb(t), Dialysierflüssigkeitsrate Qd(t) und Ultrafiltrationsrate Qf(t) bestimmt wird.

2. Verfahren nach Anspruch 1, **dadurch gekennzeichnet, daß** aus der Clearance K1 bzw. Dialysance bei der vorgegebenen Dialysierflüssigkeitsrate Qd1, Blutflußrate Qb1 und Ultrafiltrationsrate Qf1 der diffusive Anteil D1 an der Clearance bzw. Dialysance bei der vorgegebenen Dialysierflüssigkeitsrate Qd1, Blutflußrate Qb1 und Ultrafiltrationsrate Qf1 wie folgt berechnet wird:

$$D1 = \frac{K1 - Qf1}{1 - Qf1/Qe1},$$

wobei der effektive Blutfluß Qe aus dem der Förderrate der Blutpumpe (6) entsprechenden absoluten Blutfluß Qb wie folgt berechnet wird:

$$Qe = Qb\left(1 - \frac{HCT}{100}\right)Fp,$$

**6**

wobei HCT der Hämatokrit [%] und Fp die Plasmawasserfraktion ist.

3. Verfahren nach Anspruch 2, **dadurch gekennzeichnet, daß** aus der Clearance K1 bzw. Dialysance bei der vorgegebenen Dialysierflüssigkeitsrate Qd1, Blutflußrate Qb1 und Ultrafiltrationsrate Qf 1 die diffusive Dialysance D (Qd(t),Qe(t)) für beliebige Dialysierflüssigkeits-, Blutfluß- und Ultrafiltrationsraten Qd(t), Qe(t), Qf(t) nach den folgenden Gleichungen berechnet wird:

$$D(Qd(t), Qe(t)) = Qe(t) \cdot \left( 1 - \exp\left( \frac{Qd(t)}{Qd1} \ln\left( 1 - \frac{DQecorr}{Qd(t)} \right) \right) \right)$$

mit

$$DQecorr = Qd1 \left( 1 - \exp\left( \frac{Qe(t)}{Qe1} \ln\left( 1 - \frac{D1}{Qd1} \right) \right) \right).$$

4. Verfahren nach Anspruch 3, **dadurch gekennzeichnet, daß** aus der diffusiven Dialysance D(Qd(t),Qe(t) die Summe von diffusiver und konvektiver Dialysance K(Qd(t),Qe(t),Qf(t)) bzw. Clearance nach der folgenden Gleichung berechnet wird:

$$K(Qd(t), Qe(t), Qf(t)) = D((Qd(t), Qe(t)) \left( 1 - \frac{Qf(t)}{Qe(t)} \right) + Qf(t)$$

5. Verfahren nach Anspruch 4, **dadurch gekennzeichnet, daß** die Summe von diffusiver und konvektiver Dialysance K(Qd(t),Qe(t),Qf(t)) bzw. Clearance während der Dialysebehandlung für unterschiedliche Zeitpunkte t ermittelt und zur Bestimmung der effektiven Clearance $K_{eff}$ der Mittelwert gebildet wird.

6. Verfahren nach einem der Ansprüche 1 bis 5, **dadurch gekennzeichnet, daß** das Blut über einen arteriellen Zweig (5) eines extrakorporalen Kreislaufs in die Blutkammer (3) geleitet und über einen venösen Zweig (7) abgeführt wird, wobei in den arteriellen oder venösen Zweig eine Blutpumpe (6) geschaltet ist, und daß die Dialysierflüssigkeit über eine Dialysierflüssigkeitszuführleitung (9) in die Dialysierflüssigkeitskammer (4) geleitet und über eine Dialysierflüssigkeitsabführleitung (10) abgeführt wird, wobei in die Dialysierflüssigkeitszuführleitung oder Dialysierflüssigkeitsabführleitung eine Dialysierflüssigkeitspumpe (12) geschaltet ist, und daß die Bestimmung des Dialysierflüssigkeitsflusses bzw. des Blutflusses durch die Ermittlung der Förderraten der Blutpumpe (6) bzw. Dialysierflüssigkeitspumpe (12) erfolgt.

7. Dialysevorrichtung mit

   - einem Dialysator (1), der durch eine semipermeable Membran (2) in eine Blutkammer (3) und eine Dialysierflüssigkeitskammer (4) unterteilt ist,
   - einer arteriellen Blutleitung (5), die mit dem Einlaß der Blutkammer und einer venösen Blutleitung (7), die mit dem Auslaß der Blutkammer verbunden ist,
   - einer mit dem Einlaß der Dialysierflüssigkeitskammer verbundenen Dialysierflüssigkeitszuführleitung (9) und einer mit dem Auslaß der Dialysierflüssigkeitskammer verbundenen Dialysierflüssigkeitsabführleitung (10),
   - einer Einrichtung (21, 13) zur Ermittlung der Flußrate Qb des Blutes und der Dialysierflüssigkeit Qd durch die Blutkammer bzw. Dialysierflüssigkeitskammer und der Ultrafiltrationsrate Qf,
   - einer Einrichtung (21) zur Bestimmung der Clearance und/oder Dialysance bei der ermittelten Blutflussrate, Dialysierflüssigkeitsflußrate und Ultrafiltrationsrate während der Dialysebehandlung,

   **dadurch gekennzeichnet,**

**daß** die Einrichtung (21) zur Bestimmung der Clearance und/oder Dialysance eine Recheneinheit (22) zur Bestimmung der Clearance und/oder Dialysance bei der ermittelten Blutflußrate Qb(t), Dialysierflüssigkeitsrate Qd(t) und Ultrafiltrationsrate Qf(t) auf der Grundlage der sich bei einer vorgegebenen Blutflußrate Qb1, Dialysierflüssigkeitsrate Qd1 und Ultrafiltrationsrate einstellenden Clearance K1 und/oder Dialysance aufweist.

8. Dialysevorrichtung nach Anspruch 7, **dadurch gekennzeichnet, daß** aus der Clearance K1 bzw. Dialysance bei der vorgegebenen Dialysierflüssigkeitsrate Qd1, Blutflußrate Qb1 und Ultrafiltrationsrate Qf1 in der Recheneinheit der diffusive Anteil D1 an der Clearance bzw. Dialysance bei der vorgegebenen Dialysierflüssigkeitsrate Qd1, Blutflußrate Qb1 und Ultrafiltrationsrate Qf 1 wie folgt berechnet wird:

$$D1 = \frac{K1 - Qf1}{1 - Qf1/Qe1},$$

wobei der effektive Blutfluß Qe aus dem der Förderrate der Blutpumpe entsprechenden absoluten Blutfluß Qb wie folgt berechnet wird:

$$Qe = Qb\left(1 - \frac{HCT}{100}\right)Fp,$$

wobei HCT der Hämatokrit [%] und Fp die Plasmawasserfraktion ist.

9. Dialysevorrichtung nach Anspruch 8, **dadurch gekennzeichnet, daß** in der Recheneinheit aus der Clearance K1 bzw. Dialysance bei der vorgegebenen Dialysierflüssigkeitsrate Qd1, Blutflußrate Qb1 und Ultrafiltrationsrate Qf 1 die diffusive Dialysance D(Qd(t),Qe(t)) für beliebige Dialysierflüssigkeitsraten, Blutflußraten und Ultrafiltrationsraten Qd(t), Qe(t), Qf(t) nach den folgenden Gleichungen berechnet wird:

$$D(Qd(t),Qe(t)) = Qe(t) \cdot \left(1 - \exp\left(\frac{Qd(t)}{Qd1}ln\left(1 - \frac{DQecorr}{Qd(t)}\right)\right)\right)$$

mit

$$DQecorr = Qd1\left(1 - \exp\left(\frac{Qe(t)}{Qe1}ln\left(1 - \frac{D1}{Qd1}\right)\right)\right).$$

10. Dialysevorrichtung nach Anspruch 9, **dadurch gekennzeichnet, daß** in der Recheneinheit aus der diffusiven Dialysance D(Qd(t),Qe(t) die Summe von diffusiver und konvektiver Dialysance K(Qd(t),Qe(t),Qf(t)) bzw. Clearance nach der folgenden Gleichung berechnet wird:

$$K(Qd(t),Qe(t),Qf(t)) = D((Qd(t),Qe(t))\left(1 - \frac{Qf(t)}{Qe(t)}\right) + Qf(t)$$

11. Dialysevorrichtung nach einem der Ansprüche 7 bis 10, **dadurch gekennzeichnet, daß** die Einrichtung (21) zur Bestimmung der Clearance und/oder Dialysance derart ausgebildet ist, daß die Clearance und/oder Dialysance während der Dialysebehandlung für unterschiedliche Zeitpunkte t bestimmbar und zur Ermittlung der effektiven Clearance Keff der Mittelwert bestimmbar ist.

12. Dialysevorrichtung nach einem der Ansprüche 7 bis 11, **dadurch gekennzeichnet, daß** in die arterielle oder venöse Blutleitung eine Blutpumpe (6) und in die Dialysierflüssigkeitszuführleitung oder Dialysierflüssigkeitsabführleitung eine Dialysierflüssigkeitspumpe (12) geschaltet ist, wobei die Einrichtung (21) zur Ermittlung der Blutflußrate Qb(t) und/oder Dialysierflüssigkeitsrate Qd(t) die Förderrate der Blutpumpe bzw. Dialysierflüssigkeitspumpe erfaßt.

**Claims**

1. A method for determining the performance of a dialyser of a dialysis apparatus during a dialysis treatment, wherein blood flows at a preset flow rate through the blood chamber (3) of a dialyser (1) divided by a semi-permeable membrane (2) into the blood chamber and a dialysing fluid chamber (4) and dialysing fluid flows at a preset flow rate through the dialysing fluid chamber, **characterised in that**
blood flow rate Qb and dialysing fluid flow rate Qd through the blood chamber (3) and respectively the dialysing fluid chamber (4) of the dialyser (1) and ultrafiltration rate Qf are ascertained during the dialysis treatment, and that, on the basis of clearance K1 and/or dialysance becoming established at a preset blood flow rate Qb1, dialysing fluid rate Qd1 and ultrafiltration rate Qf1, clearance and/or dialysance D(Qd(t), Qe(t), Qf(t)) is determined at the ascertained blood flow rate Qb(t), dialysing fluid rate Qd(t) and ultrafiltration rate Qf(t).

2. The method according to claim 1, **characterised in that** diffusive component D1 of the clearance or dialysance at preset dialysing fluid rate Qd1, blood flow rate Qb1 and ultrafiltration rate Qf1 is calculated from clearance K1 or dialysance at preset dialysing fluid rate Qd1, blood flow rate Qb1 and ultrafiltration rate Qf1 as follows:

$$D1 = \frac{K1 - Qf1}{1 - Qf1 / Qe1},$$

wherein effective blood flow Qe is calculated from absolute blood flow Qb corresponding to the delivery rate of the blood pump (6) as follows:

$$Qe = Qb\left(1 - \frac{HCT}{100}\right)Fp,$$

wherein HCT is the haematocrit [%] and Fp the plasma water fraction.

3. The method according to claim 2, **characterised in that** diffusive dialysance D(Qd(t), Qe(t)) for arbitrary dialysing fluid, blood flow and ultrafiltration rates Qd(t), Qe(t), Qf(t) is calculated from clearance K1 or dialysance at preset dialysing fluid rate Qd1, blood flow rate Qb1 and ultrafiltration rate Qf1 according to the following equations:

$$D(Qd(t), Qe(t)) = Qe(t).\left(1 - \exp\left(\frac{Qd(t)}{Qd1}\ln\left(1 - \frac{DQecorr}{Qd(t)}\right)\right)\right)$$

with

$$DQecorr = Qd1\left(1 - \exp\left(\frac{Qe(t)}{Qe1}\ln\left(1 - \frac{D1}{Qd1}\right)\right)\right).$$

4. The method according to claim 3, **characterised in that** the sum of diffusive and convective dialysance K(Qd(t), Qe(t), Qf(t)) or clearance is calculated from diffusive dialysance D(Qd(t), Qe(t)) according to the following equation:

$$K(Qd(t), Qe(t), Qf(t)) = D((Qd(t), Qe(t))\left(1 - \frac{Qf(t)}{Qe(t)}\right) + Qf(t)$$

**5.** The method according to claim 4, **characterised in that** the sum of diffusive and convective dialysance K(Qd(t), Qe(t), Qf(t)) or clearance is ascertained during the dialysis treatment for different times t and the mean value is taken in order to determine effective clearance $K_{eff}$.

**6.** The method according to any one of claims 1 to 5, **characterised in that** the blood is conveyed via an arterial branch (5) of an extracorporeal circuit into the blood chamber (3) and carried away via a venous branch (7), whereby a blood pump (6) is incorporated into the arterial or venous branch, and that the dialysing fluid is conveyed via a dialysing fluid supply line (9) into the dialysing fluid chamber (4) and carried away via a dialysing fluid discharge line (10), whereby a dialysing fluid pump (12) is incorporated into the dialysing fluid supply line or dialysing fluid discharge line, and that the determination of the dialysing fluid flow or the blood flow takes place by ascertaining the delivery rates of the blood pump (6) or the dialysing fluid pump (12).

**7.** A dialysis apparatus with

- a dialyser (1), which is divided by a semipermeable membrane (2) into a blood chamber (3) and a dialysing fluid chamber (4),
- an arterial blood line (5) which is connected to the inlet of the blood chamber and a venous blood line (7) which is connected to the outlet of the blood chamber,
- a dialysing fluid supply line (9) connected to the inlet of the dialysing fluid chamber and a dialysing fluid discharge line (10) connected to the outlet of the dialysing fluid chamber,
- a device (21, 13) for ascertaining flow rate Qb of the blood and of the dialysing fluid Qd through the blood chamber and dialysing fluid chamber respectively and ultrafiltration rate Qf,
- a device (21) for determining the clearance and/or dialysance at the ascertained blood flow rate, dialysing fluid flow rate and ultrafiltration rate during the dialysis treatment,

**characterised in that**
the device (21) for determining the clearance and/or dialysance comprises a computing unit (22) for determining the clearance and/or dialysance at ascertained blood flow rate Qb(t), dialysing fluid rate Qd(t) and ultrafiltration rate Qf(t) on the basis of clearance K1 and/or dialysance becoming established at a preset blood flow rate Qb1, dialysing fluid rate Qd1 and ultrafiltration rate.

**8.** The dialysis apparatus according to claim 7, **characterised in that** diffusive component D1 of the clearance or dialysance at preset dialysing fluid rate Qd1, blood flow rate Qb1 and ultrafiltration rate Qf1 is calculated in the computing unit from clearance K1 or dialysance at preset dialysing fluid rate Qd1, blood flow rate Qb1 and ultrafiltration rate Qf1 as follows:

$$D1 = \frac{K1 - Qf1}{1 - Qf1/Qe1},$$

wherein effective blood flow Qe is calculated from absolute blood flow Qb corresponding to the delivery rate of the blood pump as follows:

$$Qe = Qb\left(1 - \frac{HCT}{100}\right)Fp,$$

wherein HCT is the haematocrit [%] and Fp the plasma water fraction.

**9.** The dialysis apparatus according to claim 8, **characterised in that** diffusive dialysance D(Qd(t), Qe(t)) for arbitrary dialysing fluid rates, blood flow rates and ultrafiltration rates Qd(t), Qe(t), Qf(t) is calculated in the computing unit from clearance K1 or dialysance at preset dialysing fluid rate Qd1, blood flow rate Qb1 and ultrafiltration rate Qf1 according to the following equations:

$$D(Qd(t), Qe(t)) = Qe(t) \cdot \left( 1 - \exp\left( \frac{Qd(t)}{Qd1} ln\left( 1 - \frac{DQecorr}{Qd(t)} \right) \right) \right)$$

with

$$DQecorr = Qd1\left( 1 - \exp\left( \frac{Qe(t)}{Qe1} ln\left( 1 - \frac{D1}{Qd1} \right) \right) \right).$$

**10.** The dialysis apparatus according to claim 9, **characterised in that** the sum of diffusive and convective dialysance K(Qd(t), Qe(t), Qf(t)) or clearance is calculated in the computing unit from diffusive dialysance D(Qd(t), Qe(t)) according to the following equation:

$$K(Qd(t), Qe(t), Qf(t)) = D((Qd(t), Qe(t))\left( 1 - \frac{Qf(t)}{Qe(t)} \right) + Qf(t)$$

**11.** The dialysis apparatus according to any one of claims 7 to 10, **characterised in that** the device (21) for determining the clearance and/or dialysance is designed in such a way that the clearance and/or dialysance can be determined during the dialysis treatment for different times t and the mean value can be determined in order to ascertain effective clearance $K_{eff}$.

**12.** The dialysis apparatus according to any one of claims 7 to 11, **characterised in that** a blood pump (6) is incorporated into the arterial or venous blood line and a dialysing fluid pump (12) is incorporated into the dialysing fluid supply line or dialysing fluid discharge line, whereby the device (21) for ascertaining blood flow rate Qb(t) and/or dialysing fluid rate Qd(t) detects the delivery rate of the blood pump and dialysing fluid pump respectively.

**Revendications**

**1.** Procédé destiné à déterminer la performance d'un dialyseur d'un dispositif de dialyse pendant une séance de dialyse, dans lequel le sang circule avec un débit prédéfini à travers le compartiment à sang (3) d'un dialyseur (1) séparé par une membrane (2) semi-perméable en un compartiment à sang (3) et un compartiment à liquide de dialyse (4), et le liquide de dialyse circule avec un débit prédéfini à travers le compartiment à liquide de dialyse, **caractérisé en ce que** le débit du sang Qb et le débit du liquide de dialyse Qd à travers le compartiment à sang (3) ou le compartiment à liquide de dialyse (4) du dialyseur (1) et le débit d'ultrafiltration Qf sont déterminés pendant la séance de dialyse, et **en ce que** la clairance et/ou la dialysance D(Qd(t), Qe(t), Qf(t)) en présence du débit du sang Qb(t), du débit du liquide de dialyse Qd(t) et du débit d'ultrafiltration Qf(t) calculés est déterminée sur la base de la clairance K1 et/ou de la dialysance se régulant avec un débit du sang Qb1, un débit du liquide de dialyse Qd1 et un débit d'ultrafiltration Qf1 prédéfinis.

**2.** Procédé selon la revendication 1, **caractérisé en ce que,** à partir de la clairance K1 ou de la dialysance en présence d'un débit du liquide de dialyse Qd1, d'un débit du sang Qb1 et d'un débit d'ultrafiltration Qf1 prédéfinis, la partie diffusive D1 de la clairance ou de la dialysance, en présence d'un débit du liquide de dialyse Qd1, d'un débit du sang Qb1 et d'un débit d'ultrafiltration Qf1 prédéfinis, est calculée de la manière suivante :

EP 1 062 960 B1

$$D1 = \frac{K1 - Qf1}{1 - Qf1/Qe1} \quad ,$$

le flux sanguin effectif Qe étant calculé à partir du flux sanguin absolu Qb, correspondant à la vitesse de refoulement de la pompe sanguine (6), de la manière suivante :

$$Qe = Qb\left(1 - \frac{HCT}{100}\right)Fp,$$

HCT étant l'hématocrite [%] et Fp la fraction eau du plasma.

**3.** Procédé selon la revendication 2, **caractérisé en ce que,** à partir de la clairance K1 ou de la dialysance en présence d'un débit du liquide de dialyse Qd1, d'un débit du sang Qb1 et d'un débit d'ultrafiltration Qf1 prédéfinis, la dialysance diffusive D(Qd(t), Qe(t)) pour n'importe quel débit du liquide de dialyse, du sang et de l'ultrafiltration Qd(t), Qe(t), Qf(t) est calculée selon les équations suivantes :

$$D(Qd(t), Qe(t)) = Qe(t) \cdot \left(1 - \exp\left(\frac{Qd(t)}{Qd1} ln\left(1 - \frac{DQecorr}{Qd(t)}\right)\right)\right)$$

avec

$$DQecorr = Qd1\left(1 - \exp\left(\frac{Qe(t)}{Qe1} ln\left(1 - \frac{D1}{Qd1}\right)\right)\right).$$

**4.** Procédé selon la revendication 3, **caractérisé en ce que**, à partir de la dialysance diffusive D(Qd(t), Qe(t)), la somme de la dialysance K(Qd(t), Qe(t), Qf(t)) ou de la clairance diffusives et convectives est calculée selon l'équation suivante :

$$K(Qd(t), Qe(t), Qf(t)) = D((Qd(t), Qe(t))\left(1 - \frac{Qf(t)}{Qe(t)}\right) + Qf(t)$$

**5.** Procédé selon la revendication 4, **caractérisé en ce que** la somme de la dialysance K(Qd(t), Qe(t), Qf(t)) ou de la clairance diffusives et convectives est calculée pendant la séance de dialyse à différents instants t et la valeur moyenne est formée pour déterminer la clairance effective $K_{eff}$.

**6.** Procédé selon l'une quelconque des revendications 1 à 5, **caractérisé en ce que** le sang est introduit dans le compartiment à sang (3) via une ligne artérielle (5) d'un circuit extra-corporel et est évacué via une ligne veineuse (7), une pompe à sang (6) étant montée dans la ligne artérielle ou la ligne veineuse, et **en ce que** le liquide de dialyse est introduit dans le compartiment à liquide de dialyse (4) via une ligne d'admission (9) et est évacué via une ligne d'évacuation (10), une pompe à liquide de dialyse (12) étant montée dans la conduite d'admission ou la conduite d'évacuation, et **en ce que** le flux du liquide de dialyse ou le flux sanguin est déterminé par le calcul des vitesses de refoulement de la pompe à sang (6) ou de la pompe à liquide de dialyse (12).

**7.** Dispositif de dialyse comportant

   ♦ un dialyseur (1) séparé par une membrane (2) semi-perméable en un compartiment à sang (3) et un compartiment à liquide de dialyse (4),
   ♦ une ligne à sang artérielle (5), qui est reliée à l'entrée du compartiment à sang, et une ligne à sang veineuse (7) qui est reliée à la sortie du compartiment à sang,

♦ une ligne d'admission (9) du liquide de dialyse qui est reliée à l'entrée du compartiment à liquide de dialyse, et une ligne d'évacuation (10) du liquide de dialyse qui est reliée à la sortie du compartiment à liquide de dialyse,

♦ un dispositif (21, 13) destiné à calculer le débit Qb du sang et le débit Qd du liquide de dialyse à travers le compartiment à sang ou le compartiment à liquide de dialyse, et le débit d'ultrafiltration Qf,

♦ un dispositif (21) destiné à déterminer la clairance et/ou la dialysance en présence du débit du sang, du liquide de dialyse et d'ultrafiltration calculés pendant la séance de dialyse,

**caractérisé en ce que** le dispositif (21) destiné à déterminer la clairance et/ou la dialysance comporte une unité de calcul (22) destinée à déterminer la clairance et/ou la dialysance en présence du débit du sang Qb(t), du débit du liquide de dialyse Qd(t) et du débit d'ultrafiltration Qf(t) calculés, sur la base de la clairance K1 et/ou de la dialysance se régulant avec un débit du sang Qb1, un débit du liquide de dialyse Qd1 et un débit d'ultrafiltration Qf1 prédéfinis.

8.  Dispositif de dialyse selon la revendication 7, **caractérisé en ce que**, à partir de la clairance K1 ou de la dialysance en présence d'un débit du liquide de dialyse Qd1, d'un débit du sang Qb1 et d'un débit d'ultrafiltration Qf1 prédéfinis, la partie diffusive D1 de la clairance ou de la dialysance, en présence d'un débit du liquide de dialyse Qd1, d'un débit du sang Qb1 et d'un débit d'ultrafiltration Qf1 prédéfinis, est calculée dans l'unité de calcul de la manière suivante :

$$D1 = \frac{K1 - Qf1}{1 - Qf1/Qe1} \ ,$$

le flux sanguin effectif Qe étant calculé à partir du flux sanguin absolu Qb, correspondant à la vitesse de refoulement de la pompe à sang (6), de la manière suivante :

$$Qe = Qb\left(1 - \frac{HCT}{100}\right)Fp,$$

HCT étant l'hématocrite [%] et Fp la fraction eau du plasma.

9.  Dispositif de dialyse selon la revendication 8, **caractérisé en ce que**, à partir de la clairance K1 ou de la dialysance en présence d'un débit du liquide de dialyse Qd1, d'un débit du sang Qb1 et d'un débit d'ultrafiltration Qf1 prédéfinis, la dialysance diffusive D(Qd(t), Qe(t)) pour n'importe quel débit du liquide de dialyse, du sang et de l'ultrafiltration Qd(t), Qe(t), Qf(t) est calculée dans l'unité de calcul selon les équations suivantes :

$$D(Qd(t),Qe(t)) = Qe(t) \cdot \left(1 - \exp\left(\frac{Qd(t)}{Qd1} ln\left(1 - \frac{DQecorr}{Qd(t)}\right)\right)\right)$$

avec

$$DQecorr = Qd1\left(1 - \exp\left(\frac{Qe(t)}{Qe1} ln\left(1 - \frac{D1}{Qd1}\right)\right)\right).$$

10. Dispositif de dialyse selon la revendication 9, **caractérisé en ce que**, à partir de la dialysance diffusive D(Qd(t), Qe(t)), la somme de la dialysance K(Qd(t), Qe(t), Qf(t)) ou de la clairance diffusives et convectives est calculée dans l'unité de calcul selon l'équation suivante :

$$K(Qd(t), Qe(t), Qf(t)) = D((Qd(t), Qe(t)) \left(1 - \frac{Qf(t)}{Qe(t)}\right) + Qf(t)$$

**11.** Dispositif de dialyse selon l'une quelconque des revendications 7 à 10, **caractérisé en ce que** le dispositif (21) destiné à déterminer la clairance et/ou la dialysance est réalisé de telle sorte que la clairance et/ou la dialysance peuvent être déterminées pendant la séance de dialyse à différents instants t et la valeur moyenne peut être déterminée pour calculer la clairance effective $K_{eff}$.

**12.** Dispositif de dialyse selon l'une quelconque des revendications 7 à 11, **caractérisé en ce qu'**une pompe à sang (6) est montée dans la ligne à sang artérielle ou veineuse, et une pompe à liquide de dialyse (12) est montée dans la conduite d'admission ou la conduite d'évacuation du liquide de dialyse, le dispositif (21), destiné à déterminer le débit du sang Qb(t) et/ou le débit du liquide de dialyse Qd(t), enregistrant la vitesse de refoulement de la pompe à sang et de la pompe à liquide de dialyse.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 3938662 C2
- EP 0428927 A1
- DE 19739100 C1
- DE 19746367 A1
- DE 4239937 A1

**In der Beschreibung aufgeführte Nicht-Patentliteratur**

- Blutreinigungsverfahren. Georg-Thieme-Verlag, 1990, 11-13
- **SIGDELL ; TERSTEEGEN.** *Clearance of a Dialyzer Under Varying Operating Conditions,* 1986, vol. 10 (3), 219-225